# EUROPEAN PATENT SPECIFICATION

(11) **EP 0 659 403 B1**
(45) Date of publication and mention of the grant of the patent: **14.03.2001**
(21) Application number: 94118881.5
(22) Date of filing: 30.11.1994
(51) Int. Cl.: A61K 7/48, A61K 9/18

(54) **Use of oil adsorbent natural polymer for cosmetic and pharmaceutical applications**
Verwendung von Öl adsorbierenden natürlichen Polymeren in kosmetischen und pharmazeutischen Anwendungen
Utilisation de polymère naturel adsorbant l'huile dans des applications cosmétiques et pharmaceutiques

(30) Priority: 15.12.1993 US 167388
(43) Date of publication of application: 28.06.1995
(73) Proprietor: National Starch and Chemical Investment Holding Corporation, Wilmington, Delaware 19809 (US)
(72) Inventor: Host, Sherilee J., North Tarrytown, New York, 10591 (US); Tsai, John, Belle Mead, New Jersey 08502 (US)
(74) Representative: Hagemann, Heinrich, Dr.rer.nat., Dipl.-Chem.

(56) References cited:
- EP-A- 0 182 296
- WO-A-93/17784
- US-A- 2 797 201
- US-A- 3 499 962
- US-A- 4 232 052
- US-A- 4 755 397

## Description

This invention relates to the use of an oil absorbent natural polymer in the preparation of cosmetics and pharmaceuticals as a carrier for drugs, additives, binders and pigments. More particularly, this invention relates to the use of highly absorbent starch that has been spray dried in the presence of a latent gas and that is in the form of minute spheres having an open hollow cavity.

Many of the active ingredients for pharmaceuticals, cosmetics, or agricultural chemicals are oils, semisolid fats, or oil soluble compounds. Conventional cosmetics, for example, may contain vegetable, animal and mineral oils, fats, waxes, esters, coloring agents, fragrances, preservatives, antioxidants, and sunscreens, all of which may be oils or oil soluble. Likewise, in the field of pharmaceuticals, many active ingredients, such as drugs and vitamins, are oils or are oil soluble. Historically, starch, talc, carbonates, and kaolin have been used either as essential ingredients or as charges or fillers in combination with the oils or oil soluble actives for pharmaceutical and cosmetic preparations. In the agricultural field, herbicides and pesticides are routinely combined with dry-form carriers to convert to powders for ease of handling and depositing.

The ease and efficiency of dry-form carriers makes this a preferred method of delivery for many compounds. Nevertheless, the actual physical combination of powdered and oil soluble ingredients is difficult to achieve cleanly and uniformly, and the blend often results in a composition that is not homogeneous and that is difficult to handle. In a cosmetic preparation, the result can be an oily or sticky cosmetic that lacks an acceptable skin feel. In a pharmaceutical or agricultural chemical preparation, the lack of homogeneity correlates to a deficiency in the uniformity of dose.

Blending oils, semisolid fats, or oil soluble ingredients with powders is typically accomplished by mechanically mixing the ingredients together with impellers or by a combination of spraying the oily ingredients onto powders that are simultaneously being tumbled and mixed with impellers. Spraying techniques frequently take place under conditions of elevated temperatures and pressure, conditions that can lead to loss or degradation of the oily active ingredient.

Therefore, there is still a need for a means of blending oils, semisolid fats, or oil soluble ingredients with powdered ingredients that is mechanically clean and efficient and that results in a homogeneous distribution of the oily ingredients into the powdered ingredients.

This invention is a method of homogeneously delivering an oil or an oil soluble substance into a cosmetic or pharmaceutical composition comprising: blending the oil or oil soluble substance onto a polymer derived from a starch, dextrin, or gum that has been solubilized and then spray dried in the presence of a latent gas to achieve a bulk density of about 0.1g/cm³ and a surface area of between 1-2 m²/gram and that results in the formation of minute spheres each having an open hollow cavity and ranging in size up to about 120 microns until the oil or oil soluble substance is adsorbed onto the polymer, and introducing the polymer with adsorbed oil or oil soluble substance into the cosmetic or pharmaceutical composition.

This invention is also a method of sustaining release of a cosmetically or pharmaceutically active ingredient from a cosmetic or pharmaceutical composition by delivering the active adsorbed onto the polymer to the cosmetic or pharmaceutical composition and allowing the oil or oil soluble substance to desorb from the polymer over time.

In other embodiments this invention is a cosmetic or pharmaceutical composition containing the polymer onto which has been adsorbed a cosmetically or pharmaceutically active ingredient, and the composition comprising the polymer and active ingredient.

The polymer is prepared as described in United States patent 4,232,052, which is incorporated by reference. As described in that patent, the base material for the polymers of this invention can be derived from starches, such as corn, rice, potato, sago, tapioca, waxy maize, and wheat starches; from dextrins, including the enzymatic, chemical, or heat degradation products of starch; from gums (of suitably low viscosity), such as, gum arabic, alginates, pectinate, carrageenans, carboxymethyl cellulose, hydroxypropyl cellulose, methylpropyl cellulose, and cellulose xanthate; and from gelatin, soybean protein, and zein protein. Inasmuch as the polymers are derived from naturally occurring materials, they at times will be referred to natural polymers. As will be understood, the food grade base materials are particularly suited for use with pharmaceuticals and cosmetics.

The base material is solubilized in water or an aqueous alcoholic solvent, latent gas is infused into the solution, and the solution is then spray dried according to the techniques described in United States patent 2,797,201, which is incorporated by reference. Latent gas, in this context, is deemed to mean any solid, liquid or gaseous material that can be incorporated into the solution and then converted into gas.

The resulting spray dried material will be in the form of particles containing an open hollow cavity as disclosed by electron beam magnification. The particle size will be dependent on the solids content of the starting solution and the particular spraying conditions used. Particle sizes as large as 120 microns or more can be made and specific sizes for specific uses can be obtained by manipulation of the solids levels and drying parameters, which manipulation is within the expertise of those skilled in the art. The polymer is characterized by a bulk density in the range of 0.1 gram/cm³ and a surface area of 1-2 m²/gram.

Because the polymers are based on starch, gums and dextrins, they are in general hydrophilic and will exhibit the properties of hydrophilic polymers.

The resulting polymers are useful both for their oil adsorption properties and their sustained desorption properties. The oil adsorption properties are significant for the amount of active oily substance that can be carried by the powder and for the ease and cleanliness of handling and storing the oily substance, in effect, by converting the oily substance to powder form. The amount of oil or oil soluble substance that can be adsorbed will be dependent on the nature of oil, but in general will be up to about four times the weight of the polymer. The desorption properties are significant for mitigating product syneresis and for sustained release of actives, such as, for example, fragrances in cosmetics or bath powders, insect repellants, acne medications, sunscreens, or herbicides and pesticides.

The types and kinds of oils, semisolid fats, or substances soluble in oil that can be absorbed into the polymers are exemplified, but not limited, by the following: dyes, pigments, pharmaceutical drugs, herbicides, pesticides, insect repellants, sunscreens, fragrances, vitamins, castor oil, lanolin alcohols, paraffin oils, and silicon oils.

The natural polymer and the desired oil or oil soluble substance are blended by simple admixing using conventional mixing and tumbling equipment with conventional blades. There is no need for spraying the oil onto the polymer, although the oil can be dispersed into the polymer by spraying. The polymer is so highly absorbent that simple mixing accomplishes a uniform dispersal of the active oily ingredient.

### Examples

The following examples will illustrate the oil adsorption and desorption properties of a representative natural polymer and its formulation in several cosmetic applications. The representative polymer was prepared according to the method in US patent 4,232,052 and is a cold water soluble tapioca dextrin derived from tapioca with a bulk density of 0.1 g/cm³ and a specific surface area of 1.64 m²/g.

### Desorption Properties:

To test the natural polymer's potential as a sustained-release carrier, the ability of the polymer to desorb an oil was tested against the ability of talc and a commercial starch sold under the tradename DryFlo PC (by National Starch and Chemical Company, Bridgewater, New Jersey) to desorb the same oil.

Castor oil, a moderately viscous cosmetic oil and the major ingredient of lipsticks, was stained with 0.1% by weight D&C Red 17. The castor oil and dye solution was loaded onto the test powders in the following weight ratios: 2.0 g of the solution onto 2 g of talc; 2.0 g of the solution onto 2.0 g of DryFlo starch; and 2.0 g of the solution onto 1.0 g of natural oil absorbent polymer. At this level the oil-laden talc had a creamy consistency and oil-laden DryFlo starch, a liquid consistency. The 2:1 ratio of dye solution : polymer, however, resulted in a cohesive flowing powder acceptable in cosmetic processing.

A control sample (0.125 g) of the unabsorbed dye solution, and samples (0.25g) of each of the oil-laden powders, were deposited onto equivalent areas of filter paper and the diameter measured immediately and after desorption at 2 hours, 20 hours, and 24 hours. The results are set out in Table 1 and show that the natural polymer, although loaded twice as heavily with oil and dye solution than the other powders, permitted comparatively delayed and very slow desorption. Faster initial release of the dye occurred in the other cases. This indicates that the polymer can be used in compositions where sustained release of active ingredients, which are oils or oil soluble, is desired, or in compositions where syneresis normally occurs to prevent separation of the oil from the remainder of the composition.

**Table 1**

| **Desorption of 0.1% D&C Red 17 in Castor Oil** **Desorption Diameters Over Time** | | | | |
|---|---|---|---|---|
| | initial | 2-hour | 20-hour | 24-hour |
| control | 3.5 cm | 4.6 cm | 6.8 cm | 7.2 cm |
| talc | 2.5 cm | 3.1 cm | 4.1 cm | 4.5 cm |
| DryFlo starch | 2.5 cm | 3.3 cm | 4.6 cm | 5.1 cm |
| natural polymer | 2.5 cm | 2.5 cm | 3.2 cm | 3.5 cm |

### Adsorption Properties:

A. The adsorptive capability of the natural polymer was measured for eight representative cosmetic liquids: oils, esters, silicones, fragrance oils, organic sunscreen agents, insect repellent, vitamins, and sebum. Oils, represented by a lightweight mineral oil, provide emollience. Esters, represented by octyldodecanol, provide emollience and a favorable skin feel, aid in pigment dispersion, and act as binders for pressed powder systems. Silicones, represented by a heavyweight dimethicone, impart lubricity and mildness. Perfume oils, represented by the essential oil d-limonene, provide fragrance. Organic sunscreen agents, represented by octyl methoxycinnamate, provide UV protection but also irritate the skin. The insect repellent DEET (diethyl toluamide) provides efficacy at levels exceeding 17% by weight, but is adsorbed by the skin. Vitamins, represented by vitamin E acetate, help scavenge free radicals and aid in cell rejuvenation. Sebum, synthetically composed of triolein, squalene, oleic acid and mixed sterols, may impart undesirable oiliness to the skin. It may be seen, then, that of these materials several deliver benefits when in contact with the skin, whereas others present drawbacks when in contact with the skin.

The polymer was charged into the bowl of a Hobart mixer and the various oils were mixed individually and independently into samples of the polymer with a wire whisk attachment on low speed (exception: Vitamin E acetate was manually incorporated with a spatula into the powder in a beaker). Two oil-loading endpoints were reached: the first when the loaded polymer no longer visually resembled the original unloaded polymer due to particle agglomeration and the second, when the polymer no longer adsorbed oil as indicated by surface glistening.

The results are set out in Table 2 and show that the natural polymer adsorbed roughly between three quarters to more than one times its weight in the various oils, with the exception of limonene, before losing its original appearance, and adsorbed between three to three-and-a-half to four times its weight in the various oils, with the exception of vitamin E acetate, before reaching maximum adsorption.

B. The adsorptive capability of the natural polymer was measured for d-limonene relative to other representative powders: magnesium carbonate, talc, DryFlo PC starch and Purity 21 starch (commercially available starches from National Starch and Chemical Company).

The oil was loaded onto the powders as described in A above. A first endpoint was determined when particle agglomerates formed that were not readily broken with mixing. A second endpoint was reached at maximum adsorption.

The results are set out in Table 3 and show that the polymer adsorbed about one-and-one-quarter times its weight of d-limonene before reaching the first endpoint compared to the other powders, which adsorbed approximately one-fifth their weight in oil before the first endpoint. Magnesium carbonate, however, adsorbed more oil than the polymer while maintaining parity with its original appearance. At the second endpoint, however, the polymer adsorbed approximately three-and-a-half times its weight compared to magnesium carbonate at approximately two times its weight.

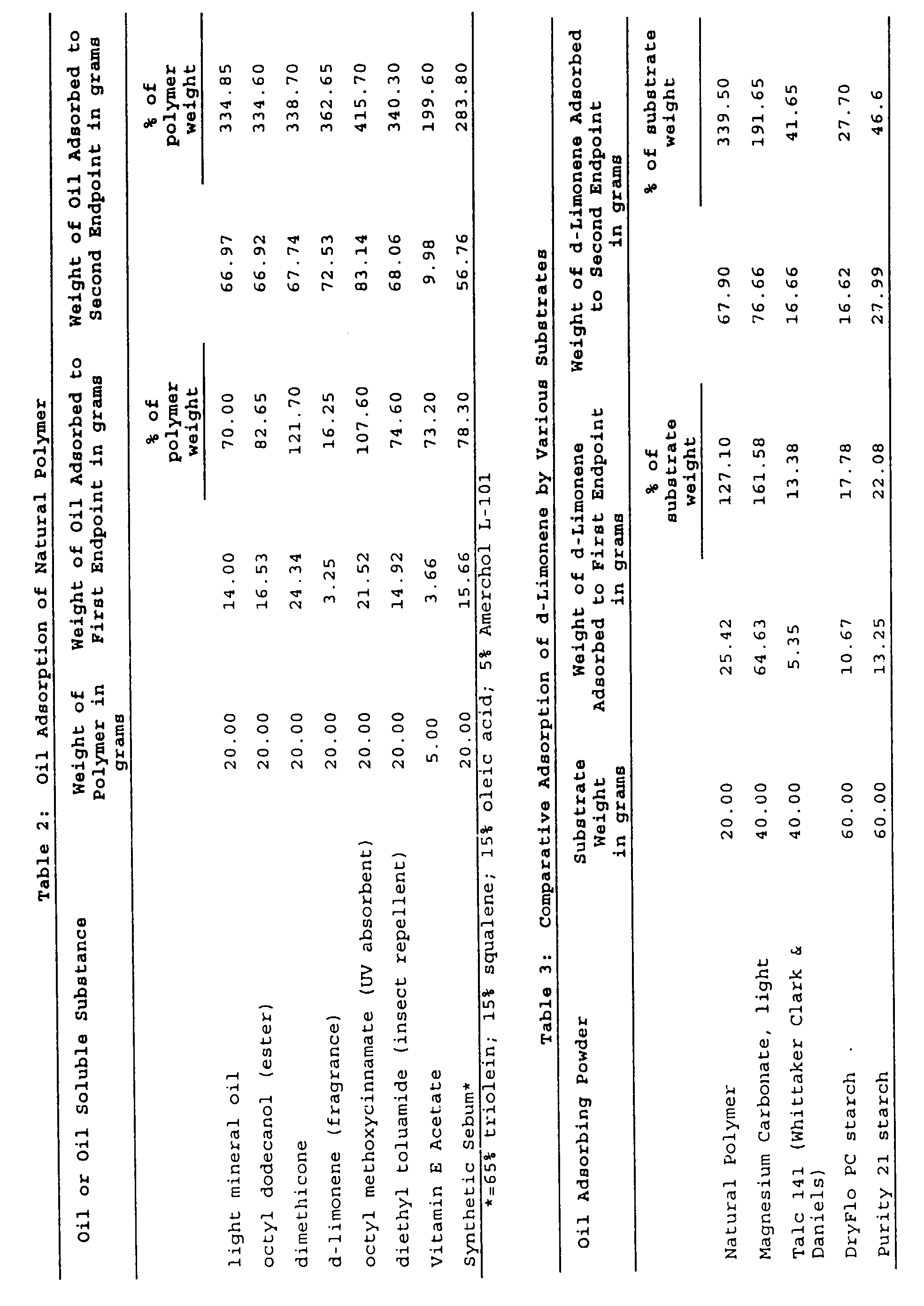

The above data show that the natural polymers may be used to introduce oils as dry materials into cosmetic formulations at greater quantities than previously used due to processing constraints, and that they can dry or degrease formulations. The data also show that the polymers will desorb more slowly than other substrates and can be used to sustain release of active ingredients, to mitigate product syneresis, and to introduce liquids evenly in powder processing without clogging screens.

These properties can also be expected to be revealed in the use of the polymers in the preparation of pharmaceuticals, agricultural chemicals, or ancillary medical uses.

### Use in Cosmetic Formulations:

The natural polymer was formulated into a loose blush and a creamy concealer and tested for oil adsorptive properties and product integrity. The formulations and test results are given below.
**Loose Blush:** The ingredients in the Table 4 were mixed in sequence to formulate a cosmetic blush: A was mixed thoroughly; B was premilled and mixed into A; AB was milled; C was blended into AB thoroughly. Control 1 had no absorbing powder, sample 2 contained DryFlo starch, and sample 3 contained natural polymer.

Specimens 10 grams each of the formulations in Table 4 were loaded with synthetic sebum until the point when the mass became a dark glistening spreadable cream. Control 1 adsorbed 6.9 grams of sebum, aporoximately 40.9% of its weight in oil; sample 2 adsorbed 5.8 grams of sebum, approximately 36.8% of its weight; and sample 3 adsorbed 8.4 grams of sebum, approximately 45.6% of its weight.

These results show that the natural polymer in a cosmetic formulation is capable of adsorbing synthetic sebum and that in actual use it will have the capability of adsorbing sebum from the skin, preventing shine breakthrough and thereby maintaining a desirable matte appearance.

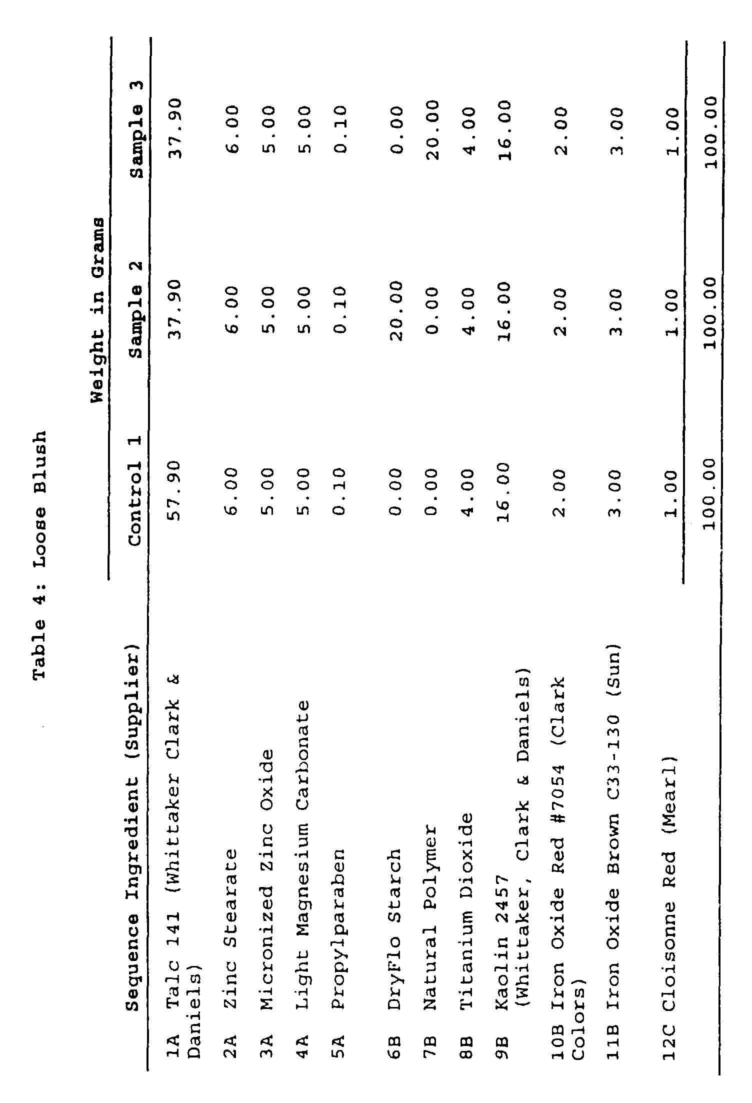

**Creamy Concealer:** The ingredients in Table 5 were mixed in sequence to formulate a cosmetic creamy concealer: A was mixed thoroughly and heated to 85°C with slow stirring; B was added and mixed into A; C was blended into AB thoroughly and ABC poured out at 65°C into pans and cylindrical tubes. Control 1 had no absorbing powder, sample 2 contained no absorbing powder and contained 5% silica, sample 3 contained DryFlo starch, and sample 4 contained natural polymer.

Control 1 was glossy and greasy in the pan and tube. Sample 2 accommodated the silica, but was still somewhat greasy. Sample 3 was less greasy than 1 but greasier than 2. Sample 4 was the least greasy and most matte in appearance. These results demonstrate that large amounts of emollients can be incorporated into a cosmetic system containing natural polymer to improve the aesthetics without yielding a greasy look.

**Table 5:**

| **Creamy Concealer** | | | | |
|---|---|---|---|---|
| | **Weight in Grams** | | | |
| **Sequence Ingredient (Supplier)** | **Control 1** | **Sample 2** | **Sample 3** | **Sample 4** |
| 1A Light Mineral Oil (Blend) | 60.84 | 55.84 | 50.84 | 50.84 |
| 2A Multiwax W-835 (Witco) | 3.15 | 3.15 | 3.15 | 3.15 |
| 3A White Beeswax | 1.35 | 1.35 | 1.35 | 1.35 |
| 4A Carnauba Wax | 3.60 | 3.60 | 3.60 | 3.60 |
| 5A BHA | 0.10 | 0.10 | 0.10 | 0.10 |
| | | | | |
| 6B Cab-O-Sil M-5 Silica (Cabot Corp.) | 0.00 | 5.00 | 0.00 | 0.00 |
| 7B DryFlo PC Starch | 0.00 | 0.00 | 10.00 | 0.00 |
| 8B Natural Polymer | 0.00 | 0.00 | 0.00 | 10.00 |
| | | | | |
| 9C Titanium Dioxide | 27.00 | 27.00 | 27.00 | 27.00 |
| 10C C33-115 Brown Iron Oxide (Sun) | 0.90 | 0.90 | 0.90 | 0.90 |
| 11C C33-130 Brown Iron Oxide (Sun) | 3.06 | 3.06 | 3.06 | . 3.06 |
| | 100.00 | 100.00 | 100.00 | 100.00 |
| Procedure: Combine A; heat to 85°C with slow mixing. Add B, mix; add C. Pour @ 65°C. | | | | |
| **Creamy Concealer:** The ingredients in Table 6 were mixed in sequence to formulate a cosmetic creamy concealer as described in the previous example. | | | | |

The different samples were then tested for integrity using a Stevens-LFRA texture analyzer at normal cycle. The test consists of measuring the force in g/cm² required to insert a cone shaped probe into the sample to a depth of 2 mm at a speed of 0.5 mm/sec. The results are set out in Table 7 and show that the samples containing the natural polymer had the higher values, indicating higher integrity. Thus, the natural polymer can be used to add a degree of hardness to normally soft cosmetic formulations.

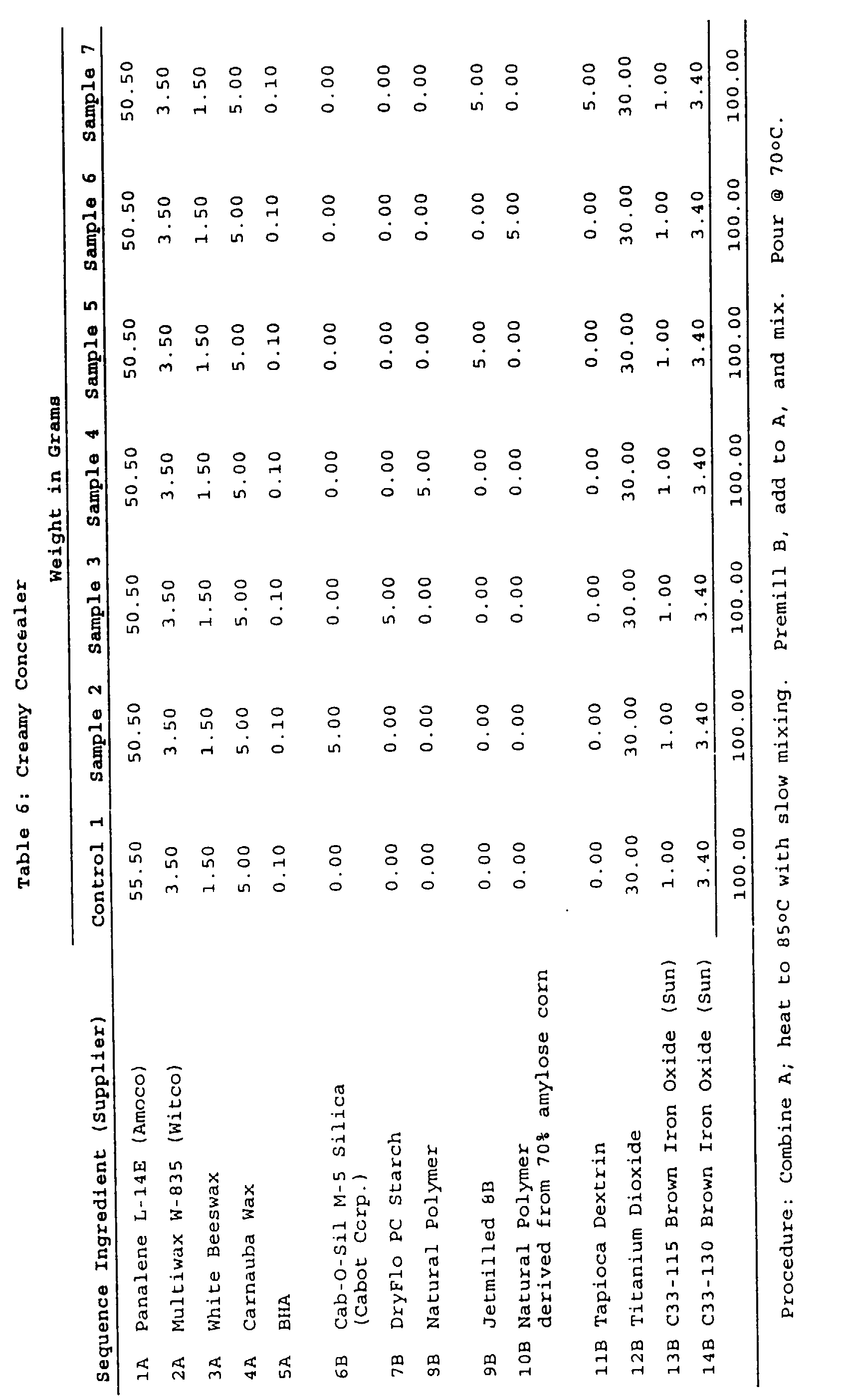

**Table 7**

| **Texture Analysis on Stevens-LFRA** | | | | | | | |
|---|---|---|---|---|---|---|---|
| **Sample** | **1** | **2** | **3** | **4** | **5** | **6** | **7** |
| **gm/cm**^{**2**} | **191** | **253** | **220** | **266** | **260** | **236** | **205** |

### Cosmetic Formulations:

Sample cosmetic compositions may be prepared according to the following formulations.

| **Pressed Powder Eyeshadow** | |
|---|---|
| **Sequence Ingredient (Supplier)** | **Weight in Grams** |
| 1A Talc 141 (Whittaker Clark & Daniels) | 37.40 |
| 2A Zinc Stearate | 5.00 |
| 3A Methylparaben | 0.20 |
| 4A Propylparaben | 0.10 |
| | 4.00 |
| 5B Titanium Dioxide | |
| 6B Iron Oxide Brown C33-115 (Sun) | 8.00 |
| 7B Iron Oxide Brown C33-130 (Sun) | 1.00 |
| | |
| 8C Natural Polymer | 10.00 |
| 9C Octyl Palmitate | 7.00 |
| | 20.00 |
| 10D Bismuth Oxychloride (Rona) | |
| 11D Colorona Bronze (Rona) | 10.00 |
| | 100.00 |
| Procedure: Combine and mix A; premill B and mix into A. Backweigh 9C slowly into 8C while mixing. Mix C into AB slowly. Premix D, mix into ABC to uniformity. Fill and press. | |

| **Pressed Face Powder** | |
|---|---|
| **Sequence Ingredient (Supplier)** | **Weight in Grams** |
| 1A Talc 141 (Whittaker Clark & Daniels) | 79.70 |
| 2A Zinc Stearate | 5.00 |
| 3A Methylparaben | 0.20 |
| 4A Propylparaben | 0.10 |
| | |
| 5B Bismuth Oxychloride (Rona) | 15.00 |
| 6B Titanium Dioxide | 5.00 |
| 7B Transparent Iron Oxide Red | 1.80 |
| 8B Transparent Iron Oxide Yellow | 4.00 |
| 9B Transparent Iron Oxide Black | 0.20 |
| | |
| 10C Natural Polymer | 25.00 |
| 11C Octyl Palmitate | 10.00 |
| | 100.00 |
| Procedure: Mix A to uniformity. Premill B and add to A. Slowly mix 11C into 10C to uniformity. Mix C into AB slowly. Mill ABC. Fill and press. | |

| **Loose Face Powder** | |
|---|---|
| **Sequence Ingredient (Supplier)** | **Weight in Grams** |
| 1A Talc 141 (Whittaker Clark & Daniels) | 79.70 |
| 2A Zinc Stearate | 5.00 |
| 3A Methylparaben | 0.20 |
| 4A Propylparaben | 0.10 |
| 5A Bismuth Oxychloride | 15.00 |
| | |
| 6B Titanium Dioxide TA-100 (Kobo) | 5.00 |
| 7B Transparent Iron Oxide Red (Rona) | 1.80 |
| 8B Transparent Iron Oxide Yellow (Rona) | 4.00 |
| 9B Transparent Iron Oxide Black (Rona) | 0.20 |
| | |
| 10C Natural Polymer | 30.00 |
| 11C Panalene L-14E (Amoco) | 10.00 |
| | 100.00 |
| Procedure: Mix A. Premill B and add to A. Slowly mix 11C into 10C to uniformity. Add C to AB slowly. Mill ABC. Fill. | |

| **Pressed Powder Blush** | |
|---|---|
| **Sequence Ingredient (Supplier)** | **Weight in Grams** |
| 1A Talc 141 (Whittaker Clark & Daniels) | 79.70 |
| 2A Zinc Stearate | 5.00 |
| 3A Methylparaben | 0.20 |
| 4A Propylparaben | 0.10 |
| 5A Bismuth Oxychloride (Rona) | 15.00 |
| 6A Mica | 15.00 |
| | |
| 7B Titanium Dioxide | 5.00 |
| 8B Iron Oxide Red | 4.00 |
| 9B Iron Oxide Yellow | 1.20 |
| 10B Iron Oxide Black | 0.20 |
| | |
| 11C Natural Polymer | 20.00 |
| 12C Panalene L-14E (Amoco) | 10.00 |
| | |
| 13D Colorona Carmine Red (Rona) | 10.00 |
| | 100.00 |
| Procedure: Mix A. Premill B and add to A. Slowly mix 12C into 11C to uniformity. Add C to AB slowly. Mill ABC. Mix D into ABC to uniformity. Fill and press. | |

| **Loose Blush** | |
|---|---|
| **Sequence Ingredient (Supplier)** | **Weight in Grams** |
| 1A Talc 141 (Whittaker Clark & Daniels) | 27.90 |
| 2A Zinc Stearate | 6.00 |
| 3A Micronized Zinc Oxide | 5.00 |
| 4A Light Magnesium Carbonate | 5.00 |
| 5A Propylparaben | 0.10 |
| | |
| 6B DryFlo Starch | 10.00 |
| 7B Natural Polymer | 20.00 |
| 8B Titanium Dioxide | 4.00 |
| 9B Kaolin 2457 (Whittaker Clark & Daniels) | 16.00 |
| 10B Iron Oxide Red #7054 (Clark Colors) | 2.00 |
| 11B Iron Oxide Brown C33-130 (Sun) | 3.00 |
| | |
| 12C Colorona Carmine Red (Rona) | 1.00 |
| | 100.00 |
| Procedure: Mix A thoroughly. Premill B and add to A. Mill AB. Blend C into AB thoroughly. Fill. | |

| **Bath Powder** | |
|---|---|
| **Sequence Ingredient (Supplier)** | **Weight in Grams** |
| 1A Talc 141 (Whittaker Clark & Daniels) | 72.85 |
| 2A Methylparaben | 0.10 |
| 3A Propylparaben | 0.05 |
| 4A Zinc Stearate | 3.00 |
| | |
| 5B C33-130 Brown Iron Oxide (Sun) | 1.55 |
| 6B C33-115 Brown Iron Oxide (Sun) | 0.45 |
| 7B Cab-O-Sil MS Silica (Cabot Corp.) | 0.00 |
| 8B DryFlo PC Starch | 5.00 |
| 9B Natural Polymer | 10.00 |
| | |
| 10C Dimethicone | 4.00 |
| 11C Octyl Dodecanol | 1.00 |
| 12C Panalene L-14E (Amoco) | 1.00 |
| 13C Finsolv TN (Finetex) | 1.00 |
| | 100.00 |
| Procedure: Combine A and blend to uniformity. Premill B and mix into A. Mist in C and blend to homogeneity. | |

| **Emulsion-Type Lipstick** | |
|---|---|
| **Sequence Ingredient** | **Weight in Grams** |
| 1A Castor Oil | 5.78 |
| 2A Candelila Wax | 7.50 |
| 3A Beeswax | 9.75 |
| 4A Isopropyl Lanolin Alcohol | 9.75 |
| 5A Isostearyl Alcohol | 9.10 |
| 6A Diisopropyl Adipate | 8.75 |
| 7A TLitanium Dioxide | 0.40 |
| 8A D&C Red #6 | 0.10 |
| 9A FD&C Yellow #5 | 0.20 |
| 10A Yellow Iron Oxide | 0.02 |
| 11A BHA | 0.10 |
| 12A Propylparaben | 0.10 |
| | |
| 13B DryFlo PC | 0.00 |
| 14B Jet-milled Natural Polymer | 5.00 |
| | |
| 16C Flamenco Super Gold (pigment) | 1.95 |
| 17C Gemstone Garnet (pigment) | 6.50 |
| | |
| 18D Distilled H₂O | 35.00 |
| 19E d-Limonene | 0.05 |
| | 100.00 |

## Claims

1. A method of homogeneously delivering an oil or an oil soluble substance into a cosmetic or pharmaceutical composition comprising:
blending the oil or oil soluble substance onto a polymer derived from a starch, dextrin, or gum that has been solubilized and then spray dried in the presence of a latent gas to achieve a bulk density of about 0.1g/cm³ and a surface area of between 1-2 m²/gram and that results in the formation of minute spheres having an open hollow cavity and ranging in size up to about 120 microns until the oil or oil soluble substance is adsorbed onto the polymer, and
introducing the polymer with adsorbed oil or oil soluble substance into the cosmetic or pharmaceutical composition.

2. The method according to claim 1 in which the oil or oil soluble substance is blended into the polymer at a level up to about four times the weight of the polymer.

3. A method of sustaining release of an oil or oil soluble active ingredient in a cosmetic or pharmaceutical composition comprising delivering to the cosmetic or pharmaceutical composition the oil or oil soluble ingredient adsorbed onto a polymer derived from a starch, dextrin, or gum that has been solubilized and then spray dried in the presence of a latent gas to achieve a bulk density of about 0.1g/cm³ and a surface area of between 1-2 m²/gram and that results in the formation of minute spheres having an open hollow cavity and ranging in size up to about 120 microns wherein the oil or oil soluble substance desorbs from the polymer over time.

4. A composition for use in cosmetics or pharmaceuticals comprising a polymer derived from a starch, dextrin, or gum that has been solubilized and then spray dried in the presence of a latent gas to achieve a bulk density of about 0.1g/cm³ and a surface area of between 1-2 m²/gram and that results in the formation of minute spheres having an open hollow cavity and ranging in size up to about 120 microns onto which has been adsorbed an oil or an oil soluble active ingredient.

5. The composition according to claim 4 in which the active ingredient is selected from the group consisting of dyes, pigments, pharmaceutical drugs, herbicides, pesticides, insect repellants, sunscreens, fragrances, vitamins, castor oil, lanolin alcohols, paraffin oils, and silicon oils.

6. The composition according to claim 4 in which the active ingredient is adsorbed onto the polymer at a level up to about four times the weight of the polymer.

7. A composition containing an oil or oil soluble cosmetically or pharmaceutically active ingredient adsorbed onto a polymer derived from a starch, dextrin, or gum that has been solubilized and then spray dried in the presence of a latent gas to achieve a bulk density of about 0.1g/cm³ and a surface area of between 1-2 m²/gram and that results in the formation of minute spheres having an open hollow cavity and ranging in size up to about 120 microns.

8. The composition according to claim 7 in which the active ingredient is selected from the group consisting of dyes, pigments, pharmaceutical drugs, herbicides, pesticides, insect repellants, sunscreens, fragrances, vitamins, castor oil, lanolin alcohols, paraffin oils, and silicon oils.

## Patentansprüche

1. Verfahren zum homogenen Abgeben eines Öls oder einer öllöslichen Substanz in eine kosmetische oder pharmazeutische Zusammensetzung, umfassend:
Mischen des Öls oder der öllöslichen Substanz auf ein Polymer, das von einer Stärke, einem Dextrin oder einem Gummi abgeleitet ist, welche(s) löslich gemacht worden und dann in Gegenwart eines latenten Gases sprühgetrocknet worden ist, um eine Schüttdichte von etwa 0,1 g/cm³ und eine Oberfläche von 1-2 m²/g zu erzielen, was zur Bildung winziger Kügelchen mit einer offenen hohlen Mulde und mit einer Größe von bis zu 120 Mikron führt, bis das Öl oder die öllösliche Substanz auf das Polymer adsorbiert ist, und
Einführen des Polymers mit dem adsorbierten Öl oder der öllöslichen Substanz in die kosmetische oder pharmazeutische Zusammensetzung.

2. Verfahren nach Anspruch 1, bei welchem das Öl oder die öllösliche Substanz in das Polymer in einer Menge von bis zum etwa Vierfachen des Gewichts des Polymers eingemischt wird.

3. Verfahren zum Verzögern der Freisetzung eines Öls oder öllöslichen aktiven Bestandteils in einer kosmetischen oder pharmazeutischen Zusammensetzung, umfassend das Abgeben an die kosmetische oder pharmazeutische Zusammensetzung des Öls oder des öllöslichen Bestandteils, adsorbiert auf einem Polymer, das von einer Stärke, einem Dextrin oder einem Gummi abgeleitet wird, welche(s) löslich gemacht worden und dann in Gegenwart eines latenten Gases sprühgetrocknet worden ist, um eine Schüttdichte von etwa 0,1 g/cm³ und eine Oberfläche von 1-2 m²/g zu erzielen, was zur Bildung winziger Kügelchen mit einer offenen hohlen Mulde und mit einer Größe von bis zu etwa 120 Mikron führt, worin das Öl oder die öllösliche Substanz im Lauf der Zeit aus dem Polymer desorbiert.

4. Zusammensetzung zur Verwendung in Kosmetika oder Pharmazeutika, umfassend ein Polymer, abgeleitet von einer Stärke, einem Dextrin oder einem Gummi, welche(s) löslich gemacht worden und dann in Gegenwart eines latenten Gases sprühgetrocknet worden ist, um eine Schüttdichte von etwa 0,1 g/cm³ und eine Oberfläche von 1-2 m²/g zu erzielen, was zur Bildung winziger Kügelchen mit einer offenen hohlen Mulde und mit einer Größe von bis zu 120 Mikron führt, auf welche ein Öl oder ein öllöslicher aktiver Bestandteil adsorbiert worden ist.

5. Zusammensetzung nach Anspruch 4, in welcher der aktive Bestandteil aus der Gruppe ausgewählt ist, die aus Farbstoffen, Pigmenten, pharmazeutischen Arzneimitteln, Herbiziden, Pestiziden, Insektenabstoßungsmitteln, Sonnenschutzmitteln, Duftstoffen, Vitaminen, Rizinusöl, Lanolinalkoholen, Paraffinölen und Silikonölen besteht.

6. Zusammensetzung nach Anspruch 4, in welcher der aktive Bestandteil auf dem Polymer in einer Menge von bis zu etwa dem Vierfachen des Gewichts des Polymers adsorbiert ist.

7. Zusammensetzung, die ein Öl oder einen öllöslichen, kosmetisch oder pharmazeutisch aktiven Bestandteil enthält, adsorbiert auf einem Polymer, das von einer Stärke, einem Dextrin oder einem Gummi abgeleitet ist, welche(s) löslich gemacht worden und dann in Gegenwart eines latenten Gases sprühgetrocknet worden ist, um eine Schüttdichte von etwa 0,1 g/cm³ und eine Oberfläche von 1-2 m²/g zu erzielen, was zur Bildung winziger Kügelchen mit einer offenen hohlen Mulde und mit einer Größe von bis zu 120 Mikron führt.

8. Zusammensetzung nach Anspruch 7, in welcher der aktive Bestandteil aus der Gruppe ausgewählt ist, die aus Farbstoffen, Pigmenten, pharmazeutischen Arzneimitteln, Herbiziden, Pestiziden, Insektenabstoßungsmitteln, Sonnenschutzmitteln, Duftstoffen, Vitaminen, Rizinusöl, Lanolinalkoholen, Paraffinölen und Silikonölen besteht.

## Revendications

1. Procédé pour distribuer de manière homogène une huile ou une substance soluble dans l'huile dans une composition cosmétique ou pharmaceutique, comprenant les étapes consistant :
à mélanger l'huile ou la substance soluble dans l'huile à un polymère dérivé d'un amidon, d'une dextrine ou d'une gomme qui a été solubilisé et ensuite séché par pulvérisation en présence d'un gaz latent pour parvenir à une masse volumique apparente d'environ 0,1 g/cm³ et à une surface spécifique de 1 à 2 m²/gramme, avec pour résultat la formation de sphères minuscules comprenant une cavité creuse ouverte et ayant des dimensions allant jusqu'à environ 120 micromètres, jusqu'à ce que l'huile ou la substance soluble dans l'huile soit adsorbée sur le polymère, et
à introduire le polymère avec l'huile ou la substance soluble dans l'huile adsorbée dans la composition cosmétique ou pharmaceutique.

2. Procédé suivant la revendication 1, dans lequel l'huile ou la substance soluble dans l'huile est mélangée au polymère en une teneur allant jusqu'à environ 4 fois le poids du polymère.

3. Procédé pour la libération prolongée d'une huile ou d'un ingrédient actif soluble dans l'huile dans une composition cosmétique ou pharmaceutique, comprenant l'étape consistant à distribuer dans la composition cosmétique ou pharmaceutique l'huile ou l'ingrédient soluble dans l'huile adsorbé sur un polymère dérivé d'un amidon, d'une dextrine ou d'une gomme qui a été solubilisé et ensuite séché par pulvérisation en présence d'un gaz latent pour parvenir à une masse volumique apparente d'environ 0,1 g/cm³ et à une surface spécifique de 1 à 2 m²/gramme, avec pour résultat la formation de sphères minuscules comprenant une cavité creuse ouverte et ayant une dimension allant jusqu'à environ 120 micromètres ; l'huile ou la substance soluble dans l'huile subissant une désorption hors du polymère au cours du temps.

4. Composition destinée à être utilisée dans des produits cosmétiques ou pharmaceutiques, comprenant un polymère dérivé d'un amidon, d'une dextrine ou d'une gomme qui a été solubilisé et ensuite séché par pulvérisation en présence d'un gaz latent pour parvenir à une masse volumique apparente d'environ 0,1 g/cm³ et à une surface spécifique de 1 à 2 m²/gramme, avec pour résultat la formation de sphères minuscules comprenant une cavité creuse ouverte et ayant des dimensions allant jusqu'à environ 120 micromètres, sur lesquelles a été adsorbée une huile ou un ingrédient actif soluble dans l'huile.

5. Composition suivant la revendication 4, dans laquelle l'ingrédient actif est choisi dans le groupe consistant en des colorants, des pigments, des agents pharmaceutiques, des herbicides, des pesticides, des répulsifs d'insectes, des écrans solaires, des parfums, des vitamines, l'huile de ricin, des alcools dérivés de la lanoline, des huiles paraffiniques et des huiles de silicone.

6. Composition suivant la revendication 4, dans laquelle l'ingrédient actif est adsorbé sur le polymère en une teneur allant jusqu'à environ 4 fois le poids du polymère.

7. Composition contenant une huile ou un ingrédient cosmétiquement ou pharmaceutiquement actif soluble dans l'huile adsorbé sur un polymère dérivé d'un amidon, d'une dextrine ou d'une gomme qui a été solubilisé et ensuite séché par pulvérisation en présence d'un gaz latent pour parvenir à une masse volumique apparente d'environ 0,1 g/cm³ et à une surface spécifique de 1 à 2 m²/gramme, avec pour résultat la formation de sphères minuscules comprenant une cavité creuse ouverte et ayant une dimension allant jusqu'à environ 120 micromètres.

8. Composition suivant la revendication 7, dans laquelle l'ingrédient actif est choisi dans le groupe consistant en des colorants, des pigments, des agents pharmaceutiques, des herbicides, des pesticides, des répulsifs d'insectes, des écrans solaires, des parfums, des vitamines, l'huile de ricin, des alcools dérivés de la lanoline, des huiles paraffiniques et des huiles de silicone.
